# EUROPEAN PATENT SPECIFICATION

(11) **EP 3 346 012 B1**
(45) Date of publication and mention of the grant of the patent: **25.12.2019**
(21) Application number: 17150649.6
(22) Date of filing: 09.01.2017
(51) Int. Cl.: C12Q 1/02, G01N 33/569

(54) **LOW ENERGETIC SHOCK WAVES FOR GENTLE RELEASE OF MICROORGANISMS FROM BIOFILMS FOR DETECTION OF FOREIGN BODY ASSOCIATED INFECTIONS**
STOSSWELLEN MIT GERINGER ENERGIE ZUR SCHONENDEN FREISETZUNG VON MIKROORGANISMEN AUS BIOFILMEN ZUM NACHWEIS VON INFEKTIONEN IM ZUSAMMENHANG MIT FREMDKÖRPERN
ONDES DE CHOC À FAIBLE IMPACT ÉNERGÉTIQUE POUR LIBÉRER EN DOUCEUR DES MICRO-ORGANISMES DE BIOFILMS AFIN DE DÉTECTER DES INFECTIONS ASSOCIÉES À DES CORPS ÉTRANGERS

(43) Date of publication of application: 11.07.2018
(73) Proprietor: Universitätsklinikum Jena, 07743 Jena (DE)
(72) Inventor: Matziolis, Georg, 07743 Jena (DE); Röhner, Eric, 97743 Jena (DE); Löffler, Bettina, 07747 Jena (DE)
(74) Representative: Schulz Junghans Patentanwälte PartGmbB

(56) References cited:
- US-A1- 2010 021 391
- US-A1- 2015 150 463
- DIVYA PRAKASH GNANADHAS ET AL: "Successful treatment of biofilm infections using shock waves combined with antibiotic therapy", SCIENTIFIC REPORTS, vol. 5, no. 1, 1 December 2015 (2015-12-01), XP055370240, DOI: 10.1038/srep17440

## Description

### Field of invention

The present invention relates to a method for detecting foreign body associated infections.

### Background

Foreign body associated infections are a severe problem accompanying implantation of devices such as stents, endoprostheses, osteosyntheses, artificial heart valves and the like. Since the majority of those infections are caused by microorganisms, which are organized in biofilms covering the implanted device, diagnosis of the microorganisms is cumbersome or even impossible, if those microorganisms are inaccessible by joint puncture or blood sampling.

Currently, samples for diagnosis of foreign body associated infections are obtained by joint puncture (synovial fluid), or blood sampling and assessed by conventional cell culture. However, since the bacteria are rather tightly attached to the biofilm, only a fraction of them can be found in synovial fluid or blood. A detection in those samples is either not possible or only possible after extensive cultivation. Extensive cultivation on the other side bears an enhanced risk of contamination leading to false positive findings. Accordingly, conventional diagnoses are limited in both sensitivity and specificity.

An alternative to the above is an investigation of the synovial fluid or the blood sample via PCR. However, such investigation is restricted to suspected bacteria due to the need of specific primers. Also here, possible contaminations of the sample may result in false positive findings.

One alternative regarding joints relates to an arthroscopic tissue sampling, since more bacteria may be found to the sampled tissue than in the aforementioned body fluid samples.

A further alternative is, if possible, the exploration of the foreign body and replacement of exchangeable parts, e.g. inlays of hip or knee endoprostheses. The explant can be subjected to an *ex vivo* sonication, wherein the biofilm is mechanically released and bacteria are determined in the sonicate.

US 2015 150463 A1 describes a method for removing or destroying a biofilm with a laser pulse.

Gnanadhas et al., Nature Scientific Reports 5:17440 2015, shows that biofilm associated infections can be treated with a combination of antibacterial drug therapy and application of shock waves.

Based on this background, it is the objective of the present invention to provide a simple, reliable and economic method for detecting of foreign body associated infections.

### Description of the invention

The above objective is attained by a method having the features of claim 1. Preferred embodiments are stated in the dependent claims and the description below.

According thereto, a method for detecting an foreign body associated infection (biofilm infections on foreign bodies, or low-grade infection) is provided. The method comprises detecting, and optionally identifying and/or quantifying, a living microorganism in a body fluid sample *ex vivo,*
wherein the microorganism was released from a biofilm covering at least partly a foreign body in the body of the patient into said body fluid by application of extracorporeal generated shock waves to the body, and said body fluid had been in contact with said foreign body prior to being obtained as a sample.The term "foreign body" in the context of the present specification particularly refers to any object that has been implanted into a body of the subject, e.g. an permanent indwelling device. Such foreign body may be of artificial origin such as a prosthesis, a stent, an artificial graft, a bolt or the like.

The term "foreign body associated infection" in the context of the present specification particularly refers to an infection caused by a biofilm at least partly covering a foreign body. The term "shock wave" in the context of the present specification particularly refers to a type of propagating disturbance, particularly to a longitudinal pressure wave, wherein a pressure change propagates as a wave front. Particularly, shock waves are characterized by a rapid pressure change and relatively high amplitudes.

Particularly, the at least one microorganism is a living microorganism, more particular the microorganism is released into the body fluid in the living state.

A living microorganism in the context of the present specification particularly refers to a microorganism that is not significantly impaired in terms of vitality or proliferation ability, and particularly a microorganism that can be propagated in cell culture.

In certain embodiments, the microorganism is identified and/or quantified in the body fluid. In certain embodiments, the body fluid is blood, lymph, liquor or synovial fluid.

In certain embodiments, the foreign body is selected from an endoprosthesis, an osteosynthesis, an artificial heart valve, a stent or an internal fixator such as a bolt, a screw or a plate.

In certain embodiments, the microorganism is selected from genus of *Salmonella, Pseudomonas, Staphylococcus, Streptococcus,* and *Propionibacterium.*

In certain embodiments, the shock waves have an intensity in the range of 0.1 mJ*m⁻² per pulse to 1 mJ*m⁻² per pulse. In certain embodiments, the shock waves have an intensity of 0.1 mJ*m⁻² per pulse.

In certain embodiments, at least 5 pulses are applied.

In certain embodiments, between 5 pulses and 50 pulses are applied. In certain embodiments, between 10 and 20 pulses are applied.

In certain embodiments, the shock waves are generated by means of a shock wave device. Particularly, the shock wave device is contacting the body of the patient such that the extracorporeal generated shock waves propagates towards or are focused on the foreign body.

In certain embodiments, the shock wave device comprises a shock wave generator and an applicator, wherein the applicator is contacting the body of the patient such that the extracorporeal generated shock waves propagates towards or are focused on the foreign body.

In certain embodiments, the foreign body is located before the application of the extracorporeal generated shock waves. Advantageously, an accurate location of the foreign body to be examined allows an accurate application of the extracorporeal generated shock waves, particularly an accurate focusing of the shock waves on the foreign body.

In certain embodiments, the foreign body is located by an imaging technique. In certain embodiments, the imaging technique is selected from ultrasonography, computer tomography or magnet resonance tomography.

In certain embodiments, the shock wave device is connected to, attached to, or aligned with an ultrasound scanner. In certain embodiments, the target area of the shock wave device can be displayed in an ultrasound image produced by the ultrasound scanner.

In certain embodiments, the microorganism is detected and optionally identified and/or quantified by cell culture, microscopy, flow cytometry or spectroscopy.

### Short description of the figures

- Fig. 1: shows one embodiment of the method of the disclosure.
- Fig. 2: shows a shock wave device usable in the method of the disclosure.
- Fig. 3: shows an alternative shock wave device usable in the method of the disclosure.
- Fig. 4: shows the release of bacteria from a biofilm covering an artificial implant in dependence of the number of applied shock wave pulses.
- Fig. 5: shows the ratio between the count of living bacteria and the total count of bacteria in dependence of the number of applied shock wave pulses.
- Fig. 6: shows the DNA concentrations as a measure of the released bacteria in dependence of the number of applied shock wave pulses.
- Fig. 7: shows cell culture trays of a sterile control sample, of one sample before and one sample after application of shock waves.

### Examples

The present invention relates to a novel non-invasive method for detecting foreign body associated infections, which is particularly characterized by an increased sensitivity and specificity, comprising the application of extracorporeal generated shock waves to facilitate the release of living bacteria from a biofilm covering a foreign body in a patient such as prostheses or the like.

Particularly, the biofilm harboring a multitude of bacteria is partly broken such that the living bacteria are released into a body fluid surrounding the foreign body, for example, synovial fluid or blood. Thereby, the concentration of living bacteria in the aforementioned body fluid can be increased by several orders of magnitude, by which a safe, reliable and accurate detection or quantification by means of conventional cell culture is possible.

Preferably, a total of 10 pulses with about 0.1 mJ*m⁻² per pulse are applied. It could be shown that the aforementioned pulse counts and energy are particularly suitable to break up the biofilm while not compromising the vitality and/ proliferative abilities of the released bacteria or body cells affected by the applied shock waves. Furthermore, implants are advantageously not impaired by the applied shock due to their low energy.

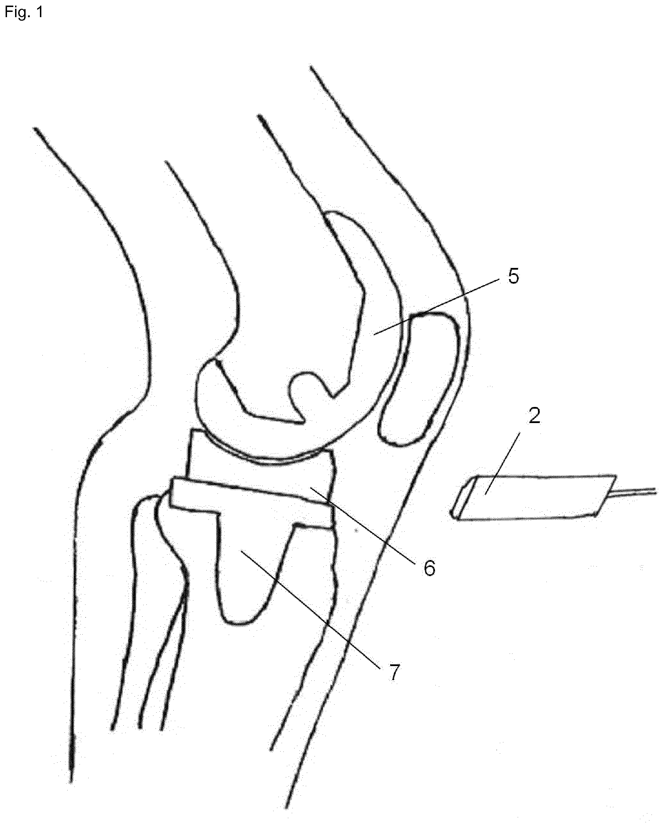

Fig. 1 illustrates a mode of applying extracorporeal generated shock waves according the method of the disclosure, wherein a shock wave device 2 is directed towards an endoprosthesis. In this example, the endoprosthesis is formed by a femur implant 5, a tibia implant 7 and an inlay 6 arranged between both aforementioned implant 5, 7. In case of such large and well locatable foreign bodies, shock waves can be applied without visual control, for example without use of additional imaging techniques such as ultrasonography.

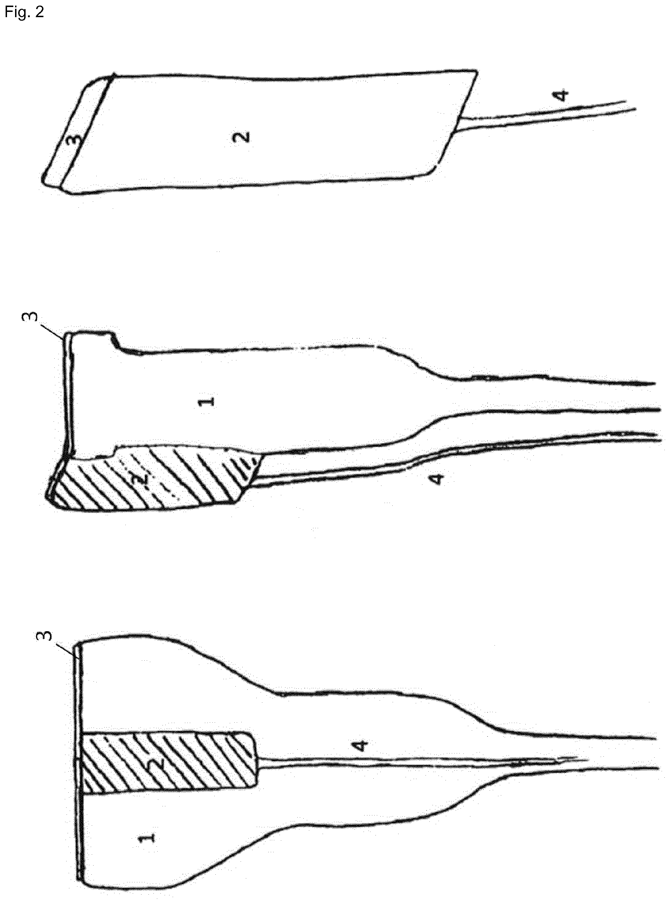

Fig. 2 illustrates an adapter or extension for conventional ultrasound probes 1, whereby a shock wave device 2 is attachable to a conventional ultrasonography equipment 1.

Preferably, the target area of the shock wave device 2 preferably can be faded into the ultrasound image, similar to the principle of interventional ultrasonography, e.g. ultrasound guided paracentesis, such that the user can located and centre the foreign body with ultrasound. Afterwards, a salvo of shock wave can be triggered, which are directed towards and able to reach the target area depicted in the ultrasound image (=located foreign body). Thereby, a precise local application of shock wave is possible, by which also small, not palpable or non-stationary objects may be addressed by the method of the disclosure.

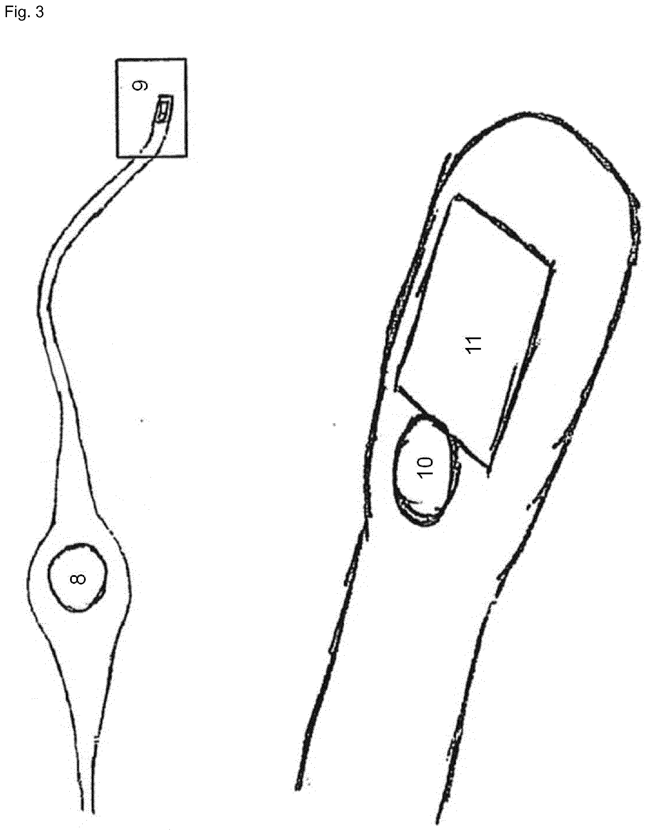

Fig. 3 shows a further embodiment of the disclosure. The depicted device corresponds to the device shown in Fig. 2. However, in this case, the ultrasound probe is configured for transesophageal sonography. Due to spatial constrains, a modular design as depicted in Fig. 2 is not feasible. Here, the shock wave device is arranged directly adjacent to the ultrasound probe and installed in the same housing. Also here, the target area can preferably be visualized by ultrasound and adjusted before a shock wave salvo is precisely released towards or applied to the foreign body (e.g. artificial heart valve).

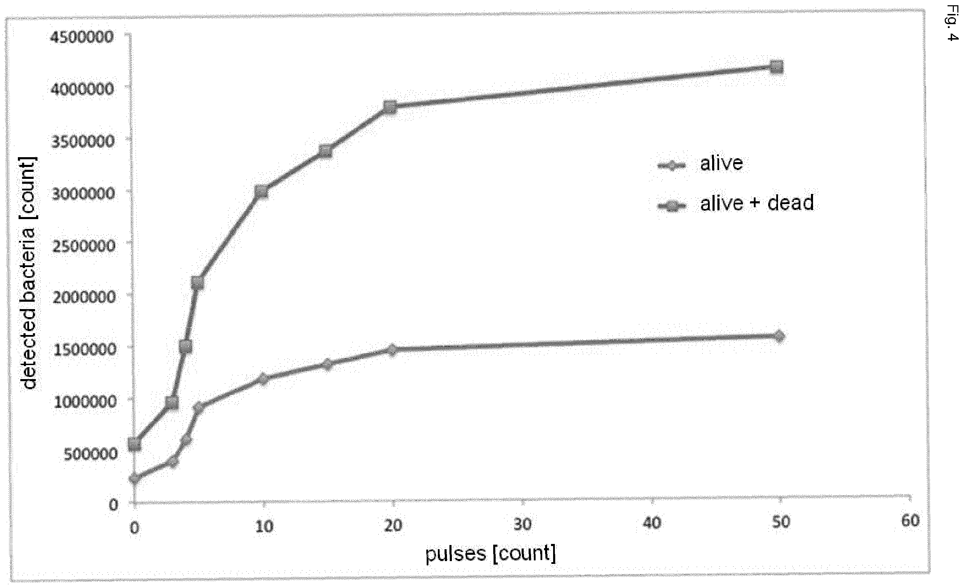

As a proof-of-concept, the dissolution of the biofilm on a plastic implant (retro patellar replacement) was investigated. The populated implant was transferred into a sterile container filled with liquid. A first sample was taken from the liquid. Afterwards, three pulses, each with 0.1 mJ*m⁻², with was applied to the container and a second sample was taken from the liquid. The procedure was continued until a total of 50 pulses were applied, wherein additional samples were taken during the procedure. Afterwards, the number of living bacteria and total count of bacteria were flowcytometrically determined (Fig. 4). A significant increase of the number of the detected bacteria with increase pulses could be observed. A minimum of 5 pulses appeared to be suitable.

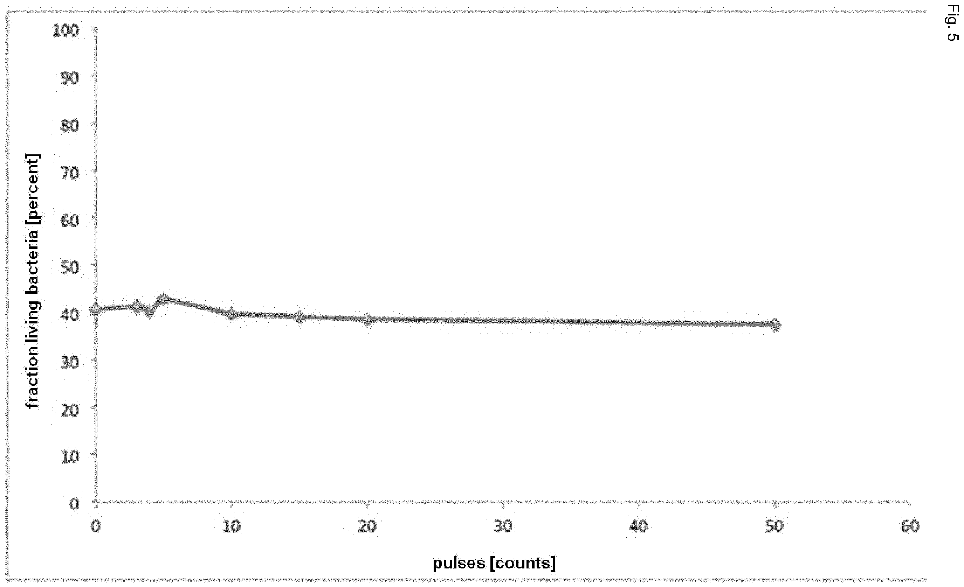

The ratio between living bacteria to the total number of bacteria remained constant in all measured sample demonstrating that the applied shock wave pulse have a bactericide effect (Fig. 5).

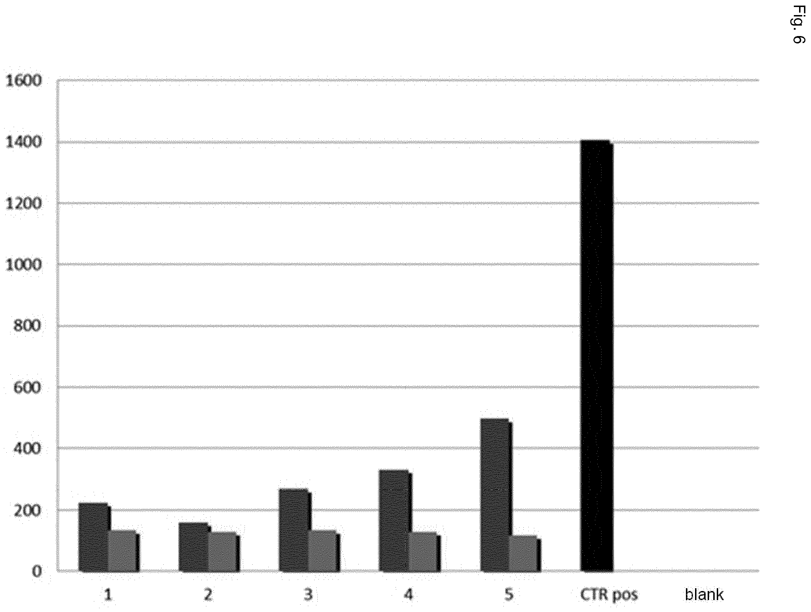

As a further confirmation of the above results, the DNA concentration in certain samples were determined. Briefly, after disruption of the cells comprised in the samples were disrupted, a fluorescent dye was added (Hoechst reagent 33258, 0.2µg/ ml, and the DNA amount was fluorometrically determined against a reference (excitation at 356 nm; emission at 458 nm) As shown in Fig. 6, also the DNA measurements shows an increase of the total number of bacteria with increasing pulses.

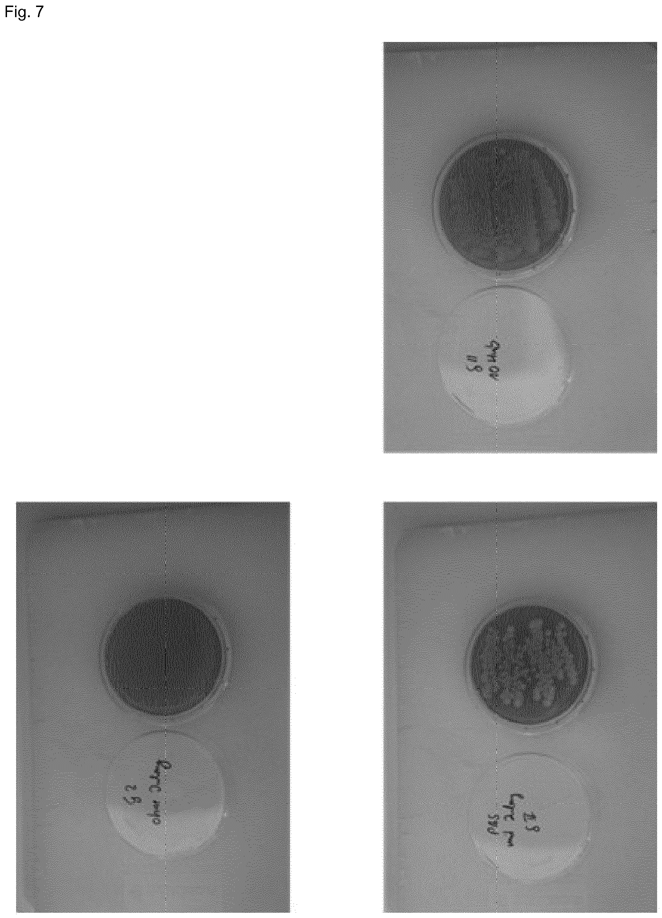

Since flowcytometry or determining of DNA concentration not necessarily are used in the clinical routine and further represent merely quantitative surrogate marker, the bacteria count was determined in accordance with the accepted clinical routine. Accordingly, the sampled bacteria cultures were determined via clinical relevant end point. Fig. 7 depicts a clinical relevant germ proof with a sterile sample as control (upper panel), the first sample before application of the pulse (left lower panel) and a sample taken after 10 pulses (right lower panel), wherein the latter shows a significant increase in germ concentration. Consequently, also clinical relevant germ proofs confirm the above data

## Claims

1. A method for detecting of a foreign-body-associated infection, comprising
- detecting, and optionally identifying and/or quantifying, a living microorganism in a body fluid sample *ex vivo,*
- wherein the microorganism was released from a biofilm covering at least partly a foreign body in the body of the patient into said body fluid by application of extracorporeal generated shock waves to the body, and said body fluid had been in contact with said foreign body prior to being obtained as a sample.

2. The method according to claim 1, **characterized in that** said body fluid is blood, lymph, liquor or synovial liquid.

3. The method according to claim 1 or 2, **characterized in that** said foreign body is an endoprosthesis, an osteosynthesis, an artificial heart valve, a stent, or an internal fixator.

4. The method according to any one of the preceding claims, **characterized in that** said microorganism is selected from the genus of *Salmonella, Pseudomonas, Staphylococcus, Streptococcus,* and *Propionibacterium.*

5. The method according to any one of the preceding claims, **characterized in that** said shock waves had an intensity in the range of 0.1 mJ*m⁻² per pulse to 1 mJ*m⁻² per pulse, particularly 0.1 mJ*m⁻² per pulse.

6. The method according to any one of the preceding claims, **characterized in that** at least 5 pulses were applied.

7. The method according to any one of the preceding claims, **characterized in that** between 5 pulses and 50 pulses were applied, preferable between 10 pulses and 20 pulses.

8. The method according to any one of the preceding claims, **characterized in that** said shock waves were generated by means of a shock wave device, wherein particularly said shock wave device is in contact to said body of said patient such that said extracorporeal generated shock waves propagates towards or are focused on said foreign body.

9. The method according to any one of the preceding claims, **characterized in that** said foreign body was located before said application of said extracorporeal generated shock waves.

10. The method according to claim 9, **characterized in that** said foreign body is located by an imaging technique.

11. The method according to claim 10, **characterized in that** said imaging technique is selected from ultrasonography, computer tomography or magnet resonance tomography.

12. The method according to any one of the preceding claims, **characterized in that** said microorganism is detected and optionally identified and/or quantified by cell culture, microscopy, flow cytometry or spectroscopy.

## Patentansprüche

1. Verfahren zum Nachweisen einer mit einem Fremdkörper verbundenen Infektion, umfassend
- Nachweisen und gegebenenfalls Identifizieren und/oder Quantifizieren eines lebenden Mikroorganismus in einer Körperflüssigkeitsprobe *ex vivo,*
- wobei der Mikroorganismus aus einem Biofilm, der zumindest teilweise einen Fremdkörper im Körper des Patienten bedeckt, durch Anwendung von extrakorporal erzeugten Stoßwellen in die Körperflüssigkeit freigesetzt wurde und die Körperflüssigkeit in Kontakt mit dem Fremdkörper war, bevor sie als Probe erhalten wurde.

2. Verfahren nach Anspruch 1, **dadurch gekennzeichnet, dass** die Körperflüssigkeit Blut, Lymphe, Liquor oder Synovialflüssigkeit ist.

3. Verfahren nach Anspruch 1 oder 2, **dadurch gekennzeichnet, dass** der Fremdkörper eine Endoprothese, eine Osteosynthese, eine künstliche Herzklappe, ein Stent oder ein Fixateur interne ist.

4. Verfahren nach einem der vorhergehenden Ansprüche, **dadurch gekennzeichnet, dass** der Mikroorganismus aus der Gattung *Salmonella, Pseudomonas, Staphylococcus, Streptococcus* und *Propionibacterium* ausgewählt ist.

5. Verfahren nach einem der vorhergehenden Ansprüche, **dadurch gekennzeichnet, dass** die Stoßwellen eine Intensität im Bereich von 0,1 mJ*m⁻² pro Puls bis 1 mJ*m⁻² pro Puls, insbesondere 0,1 mJ*m⁻² pro Puls, aufweisen.

6. Verfahren nach einem der vorhergehenden Ansprüche, **dadurch gekennzeichnet, dass** mindestens 5 Pulse angewendet wurden.

7. Verfahren nach einem der vorhergehenden Ansprüche, **dadurch gekennzeichnet, dass** zwischen 5 und 50 Pulse, vorzugsweise zwischen 10 Pulsen und 20 Pulsen angewendet wurden.

8. Verfahren nach einem der vorhergehenden Ansprüche, **dadurch gekennzeichnet, dass** die Stoßwellen mittels einer Stoßwellenvorrichtung erzeugt wurden, wobei insbesondere die Stoßwellenvorrichtung in Kontakt mit dem Körper des Patienten steht, sodass sich die extrakorporal erzeugten Stoßwellen in Richtung des Fremdkörpers ausbreiten oder auf diesen fokussiert sind.

9. Verfahren nach einem der vorhergehenden Ansprüche, **dadurch gekennzeichnet, dass** der Fremdkörper vor der Anwendung der extrakorporal erzeugten Stoßwellen lokalisiert wurde.

10. Verfahren nach Anspruch 9, **dadurch gekennzeichnet, dass** der Fremdkörper durch eine Bildgebungstechnik lokalisiert wird.

11. Verfahren nach Anspruch 10, **dadurch gekennzeichnet, dass** die Bildgebungstechnik aus Ultraschall, Computertomographie oder Magnetresonanztomographie ausgewählt ist.

12. Verfahren nach einem der vorhergehenden Ansprüche, **dadurch gekennzeichnet, dass** der Mikroorganismus durch Zellkultur, Mikroskopie, Durchflusszytometrie oder Spektroskopie nachgewiesen und gegebenenfalls identifiziert und/oder quantifiziert wird.

## Revendications

1. Procédé de détection d'une infection associée à un corps étranger, comprenant
- détecter, et éventuellement identifier et/ou quantifier, un micro-organisme vivant dans un échantillon de fluide corporel ex *vivo,*
- le micro-organisme ayant été libéré à partir d'un biofilm recouvrant au moins partiellement un corps étranger dans le corps du patient vers ledit fluide corporel par application d'ondes de choc générées extracorporelles, et ledit fluide corporel ayant été en contact avec ledit corps étranger avant d'être obtenu en tant qu'échantillon.

2. Procédé selon la revendication 1, **caractérisé par le fait que** ledit fluide corporel est le sang, la lymphe, le plasma ou le liquide synovial.

3. Procédé selon l'une des revendications 1 ou 2, **caractérisé par le fait que** ledit corps étranger est une endoprothèse, une ostéosynthèse, une valvule cardiaque artificielle, un stent ou un fixateur interne.

4. Procédé selon l'une quelconque des revendications précédentes, **caractérisé par le fait que** ledit micro-organisme est choisi parmi le genre de *Salmonella, Pseudomonas, Staphylococcus, Streptococcus* et *Propionibacterium.*

5. Procédé selon l'une quelconque des revendications précédentes, **caractérisé par le fait que** lesdites ondes de choc ont une intensité dans la plage allant de 0,1 mJ*m⁻² par impulsion à 1 mJ*m⁻² par impulsion, en particulier 0,1 mJ*m⁻² par impulsion.

6. Procédé selon l'une quelconque des revendications précédentes, **caractérisé par le fait que** au moins 5 impulsions ont été appliquées.

7. Procédé selon l'une quelconque des revendications précédentes, **caractérisé par le fait que** entre 5 impulsions et 50 impulsions ont été appliquées, de préférence entre 10 impulsions et 20 impulsions.

8. Procédé selon l'une quelconque des revendications précédentes, **caractérisé par le fait que** lesdites ondes de choc ont été générées au moyen d'un dispositif d'ondes de choc, en particulier ledit dispositif d'ondes de choc est en contact avec ledit corps dudit patient de telle sorte que lesdites ondes de choc générées extracorporelles se propagent vers ledit corps étranger ou sont concentrées sur celui-ci.

9. Procédé selon l'une quelconque des revendications précédentes, **caractérisé par le fait que** ledit corps étranger était localisé avant ladite application desdites ondes de choc générées extracorporelles.

10. Procédé selon la revendication 9, **caractérisé par le fait que** ledit corps étranger est localisé par une technique d'imagerie.

11. Procédé selon la revendication 10, **caractérisé par le fait que** ladite technique d'imagerie est choisie parmi une échographie, une tomographie par ordinateur ou une tomographie par résonance magnétique.

12. Procédé selon l'une quelconque des revendications précédentes, **caractérisé par le fait que** ledit micro-organisme est détecté et éventuellement identifié et/ou quantifié par culture cellulaire, microscopie, cytométrie en flux ou spectroscopie.
